# EUROPEAN PATENT APPLICATION

(11) **EP 2 213 241 A1**
(43) Date of publication of application: **04.08.2010**
(21) Application number: 10151395.0
(22) Date of filing: 22.01.2010
(51) Int. Cl.: A61B 8/00, A61B 8/02, G01N 29/24

(54) **Maternal and fetal monitor ultrasound transducer**

(30) Priority: 29.01.2009 US 362011
(71) Applicant: General Electric Company, Schenectady, NY 12345 (US)
(72) Inventor: Muthya, Srikanth, Odenton, MD 21113 (US); Pandit, Ashit Madhusudan, Laurel, MD 20723 (US)
(74) Representative: Illingworth-Law, William Illingworth

(57) **Abstract**

An ultrasound transducer (10) and method of producing same takes advantage of the properties of two differing materials to optimize the transducer. More specifically, the transducer (10) has one or more emitters (12) made of lead zirconate titanate (PZT) crystals (18) that are affixed to a wearable material made of polyvinylildene fluoride (PVDF) (18). As such, the PZT crystals (18) are an effective emitter of ultrasound energy into an object and/or area of interest, such as a patient for maternal and/or fetal care monitoring, while the PVDF material (22) has a low impedance and therefore effectively receives the ultrasound energy returned from the object and/or area of interest. The PVDF material (22) comprises a wearable form factor, whereby the transducer (10) utilizes different properties of different materials and combines them into a common transducer (10).

## Description

### Background

In general, the inventive arrangements relate to a transducer for ultrasound monitoring of patients and/or the like, and more specifically, to a novel ultrasound transducer that utilizes different and optimum materials for the emitter and receiver.

Electronic fetal monitoring (EFM) involves monitoring pregnant women from approximately 20 weeks of gestation through labor and delivery. It typically consists of periodic monitoring during a woman's antepartum period (i.e., during pregnancy), as well as intermittent and/or continuous monitoring during the woman's intrapartum period (i.e., during labor and delivery). In the antepartum setting, the woman may be monitored for short durations at regular intervals, while in the intrapartum setting, the woman may be monitored intermittently and/or continuously up until delivery. The former can range anywhere from approximately 20 weeks to 40 weeks, while the latter can range anywhere from approximately under 1 hour to 36 hours or more. During such times, maternal health and/or comfort is of paramount importance, as is fetal health and/or comfort.

Numerous parameters are often used to monitor both maternal and fetal well-being, including, for example, maternal electrocardiography (ECG), maternal uterine activity, maternal pulse oximetry (SpO₂), maternal blood pressure, fetal ECG, and/or fetal heart rates (FHR). FHRs, for example, are often measured in terms of the number of times a fetus's heart beats per minute (BPM), and typical ranges of FHR are between approximately 30 - 240 BPM. FHRs are usually determined in one of two ways - namely, using non-invasive ultrasound Doppler, or by using invasive fetal ECG, such as by attaching a fetal scalp electrode.

Now then, as mentioned, one common means for non-invasively monitoring an expectant mother and fetus is through the use of ultrasound. For example, FHRs can be monitored through the use of ultrasound Doppler during the woman's intrapartum period - i.e., during her labor and delivery. In such systems, ultrasonic waves are generated by a transducer that is placed against the skin of the mother, and after they are directed towards her fetus, for example, they are bounced thereagainst and returned again to the transducer. The return echoes or signals carry information obtained by a Doppler shift so as to provide a caregiver, for example, with an indication of the FHR, and such information can be usefully communicated via a heart rate monitor and/or the like.

Accordingly, one of the important components of an ultrasound transducer is an emitter, which emits ultrasound energy into a desired area of interest, such as a birthing mother and/or fetus, as well as a receiver, which receives returned ultrasound signals that are then processed and/or analyzed for pertinent patient information.

With conventional ultrasound transducers, lead zirconate titanate (PZT) crystals are commonly used for emitting and receiving ultrasound signals. However, many PZT crystals are relatively small in size, and therefore they often need to be repositioned along the abdomen of a birthing mother, for example, from one location to another location, particularly to be able to fully monitor the desired parameters of the mother and/or fetus. One of the reasons for the need to continually move the transducer is due to normal movements of the fetus, such that the transducer can lose a viable signal when the fetus moves positions and/or locations, and whereby the transducer must then be removed from the abdomen of the mother, or at least its current position thereon, and a new location searched for and located. Moving the transducer is also necessary if an expectant mother, for example, is to give birth to twins or other multiple births. In any event, while PZT crystals are good emitters of ultrasound energy, they also have a relatively high acoustic impedance and can be brittle, whereby they lack good mechanical stability.

On the other hand, polyvinylildene fluoride (PVDF) has - unlike PZT crystals - a relatively low acoustic impedance, which tends to match well with that of the bodies of patients. In addition, PVDF can be produced as a wearable material that can be worn like a fabric, whereby it can be used to cover a relatively large area of, for example, an abdomen of a patient. However, PVDF is not currently used in fetal monitoring.

Accordingly, it would be advantageous to have a novel ultrasound transducer that takes advantage of the desirable properties of both PZT crystals and PVDF materials in an optimized, combined fashion, particularly suited for maternal and/or fetal care monitoring. Summary of the Inventive Arrangements

The inventive arrangements comprise an ultrasound transducer that combines two different materials in the transducer, such that individual, advantageous properties of each material are utilized to construct an optimum transducer, particularly suited for maternal and/or fetal care monitoring.

As such, the transducer of the inventive arrangements includes an emitter comprised of one or more lead zirconate titanate (PZT) crystals, particularly since that material, when excited, is efficient in producing ultrasound energy. The transducer also includes a receiver comprising a polyvinylildene fluoride (PVDF) material, particularly adapted to receive returned ultrasound signals echoed from a desired area of interest. Having low impedance, PVDF material is particularly efficient in receiving the returned ultrasound signals.

In addition, with the PVDF material, the physical form of the material can be a wearable material, whereby it can cover, for example, a large area of an abdomen of a patient - and thus not require continually relocating the transducer to different locations about the abdomen of a pregnant, birthing, and/or near-birthing mother. Accordingly, PVDF materials are also particularly well-suited for monitoring the condition(s) of potentially birthing multiple infants, particularly as the ultrasound transducer can emit and receive ultrasound signals representative of conditions of one or more fetuses without requiring the transducer to be re-located to multiple locations about the abdomen of the wearing mother. In addition, using PVDF as a wearable material avoids the need to continually relocate the transducer due to movements of the fetus itself.

As such, the desirable properties of both PZT crystals and PVDF materials are combined into a single transducer, particularly one that takes advantage of each one's properties to provide an optimum ultrasound transducer.

Now then, these and other features and advantages of the inventive arrangements will become more readily apparent during the following detailed description taken in conjunction with the drawings herein.

### Brief Description of the Drawings

FIG. 1 is a first schematic view of a transducer constructed in accordance with the inventive arrangements; and
FIG. 2 is a second schematic view of a transducer constructed in accordance with the inventive arrangements.

### Detailed Description of Preferred Embodiments

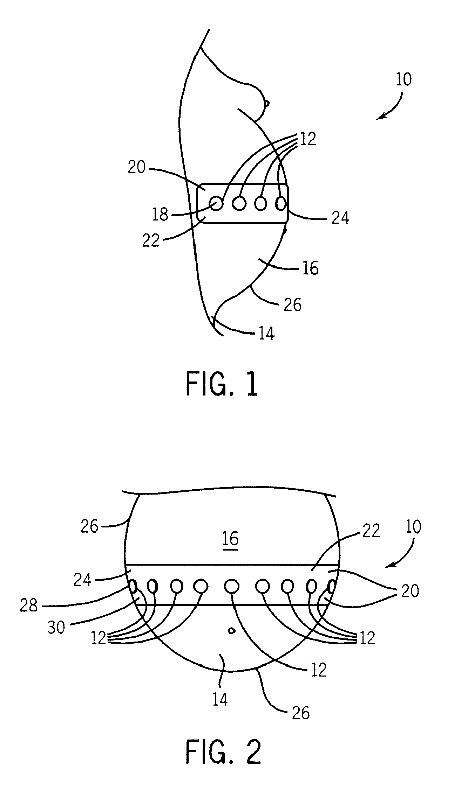

Referring now to FIG. 1, there is shown a first schematic view illustrating an ultrasound transducer 10 constructed in accordance with the inventive arrangements. More specifically, the transducer 10 includes one or more ultrasound emitters 12 (e.g., a plurality of four are representatively depicted in FIG. 1, while a plurality of eight are representatively depicted in FIG. 2), which emit ultrasound energy into a desired area of a patient 14, such as the patient's 14 abdomen 16. Ideally, the emitters 12 are comprised of one or more lead zirconate titanate (PZT) crystals 18, particularly since PZT crystals 18, when excited, are efficient in producing ultrasound energy. Indeed, they are ideally suited as an effective emitter of ultrasound energy. Through known techniques, that energy is directed towards the object of interest, such as a fetus (not shown) within the patient's 14 abdomen 16. In one embodiment, the PZT crystals 18 are configured to be excited individually and/or sequentially to emit the ultrasound energy into the patient 14, and in another or additional embodiment, they are configured to be excited simultaneously to emit the ultrasound energy into the patient 14. Accordingly, the PZT crystals 18 can be used to form a phased array about the patient's 14 abdomen 16.

The transducer 10 also includes a receiver 20 comprising a polyvinylildene fluoride (PVDF) material 22. The receiver 20 is particularly adapted to receive returned ultrasound signals echoed from the desired area of interest, such as the fetus within the patient's 14 abdomen 16. More specifically, the PVDF material 22 preferably has a relatively low acoustic impedance relative to the PZT crystals 18, which makes it particularly efficient in receiving the returned ultrasound signals. In addition, the PVDF material 22 can also be constructed as a wearable form factor that fits against the patient's 14 abdomen 16. In other words, the PVDF material 22 can be produced as a wearable material that can be worn like a fabric, whereby it can be used to cover a relatively large area of, for example, the patient's 14 abdomen 16. Having low impedance, the PVDF material 22 also allows the receiver 20 to suitably match the contours of the body of the patient 14. Accordingly, the physical form of the PVDF material 22 can be a wearable material, whereby it can cover, for example, a large area of the patient's 14 abdomen 16 - and thus not require continually relocating the transducer 10 to different locations about the abdomen 16 of, for example, a pregnant, birthing, and/or near-birthing mother. Ideally, the PVDF material 22 is configured as a band 24 that is configured to at least partially surround and/or cover the patient's 14 abdomen 16. Accordingly, the PVDF material 22 can be placed on the patient's 14 abdomen 16 such that the band 24 is formed. The band 24 thus becomes a wearable set of sensors that can be used to extract maternal and/or fetal health care parameters. It can implemented, for example, using a suitable ASIC embedded in fabric, and it allows periodic, intermittent, and/or continuous monitoring - which can also be localized and/or remotely (e.g., wirelessly) monitored and/or communicated.

In use, the PVDF material 22 is comprised of a wearable material that is placed in contact with the skin 26 of the patient 14. Ideally, the emitters 12, which emit the ultrasound energy into the desired area of the patient 14 and are ideally comprised of the PZT crystals 18, are carried by the PVDF material 22 in a suitable fashion. For example, in one embodiment, the emitters 12 are secured to the PVDF material 22 in a woven fashion.

Referring now to FIG. 2, there is shown a second schematic view of the ultrasound transducer 10, particularly shown as at least partially surrounding the patient's 14 abdomen 16. Here again the emitters 12 and PZT crystals 18 and receiver 20 and PVDF materials 22 extend in the band 24 about the patient's 14 abdomen 16, ideally making contact with the skin 26 thereof. In such an exemplary embodiment, the transducer 10 can partially and/or fully cover the patient's 14 abdomen 16, such that the transducer 10 can both emit and receive returned ultrasound signals from one or more fetuses (not shown) - that is, for example, if the birthing mother is having two or other multiple births. In such event, with the expanded coverage of the ultrasound transducer 10, there is no need to continually move individual transducers 10 to different positions along the patient's 14 abdomen 16. Ideally, the emitters 12 may also be removably placed along the receiver 20 and/or band 24, such as by a hook and loop fastening system such as Velcro attachment arrangements. Accordingly, they may be spaced about evenly by the caregiver (not shown) along a length of and/or placed randomly thereabout on the wearable portion of the PVDF material 22 so as to optimize the transmitted and received ultrasound signals. This allows the emitters 12 to be affixed about the transducer 10 and/or band 24 so as to optimize and/or achieve the best quality ultrasound signal(s).

In addition, an excitation system 28 is used to excite the emitters 12 so that the ultrasound signals are generated and/or transmitted into the patient's 14 abdomen 16. In one embodiment, the excitation system 28 excites the PZT crystals 18 individually, such as with a time delay and/or in a phased array manner, such as one after another in a defined sequence. In another embodiment, the excitation system 28 excites the PZT crystals 18 simultaneously. In addition, the return ultrasound signals are received by the receiver 20, whereby they are processed in a conventional manner and/or transmitted to a monitor 30, for example, to convey and/or display pertinent patient information.

Accordingly, a technical effect of the foregoing is to provide an improved ultrasound transducer which utilizes different properties of different materials and combines them into a common transducer, thereby improving maternal and/or fetal health care monitoring, particularly during pregnancy and/or the like.

Those skilled in the art will readily recognize that numerous adaptations and modifications can be made to the inventive arrangements and which will result in an improved ultrasound transducer and/or method for maternal and/or fetal monitoring, yet all of which will fall within the scope and spirit hereof, as defined in the following claims. Accordingly, the inventive arrangements are limited, if at all, only by the following claims and their equivalents.

Aspects of the present invention are defmed in the following numbered clauses:
1. An ultrasound transducer, comprising:
   at least one ultrasound emitter comprising one or more lead zirconate titanate (PZT) crystals adapted to emit ultrasound energy into a desired area of interest; and
   at least one ultrasound receiver comprising a polyvinylildene fluoride (PVDF) material adapted to receive returned ultrasound signals echoed from the desired area of interest.
2. The ultrasound transducer of Clause 1, wherein the PZT crystals are secured to the PVDF material.
3. The ultrasound transducer of Clause 1 or Clause 2, wherein the PZT crystals are removably secured to the PVDF material.
4. The ultrasound transducer of any one of the preceding Clauses, wherein the PZT crystals are woven into the PVDF material.
5. The ultrasound transducer of any one of the preceding Clauses, further comprising:
   an excitation system adapted to excite the PZT crystals to emit said ultrasonic energy
6. The ultrasound transducer of any one of the preceding Clauses, wherein one or more of the PZT crystals are configured to be individually excited to emit said ultrasound energy.
7. The ultrasound transducer of any one of the preceding Clauses, wherein one or more of the PZT crystals are configured to be simultaneously excited to emit said ultrasound energy.
8. The ultrasound transducer of any one of the preceding Clauses, wherein at least a part of the PVDF material is configured as a band that is configured to at least partially surround an abdomen of a person.
9. The ultrasound transducer of any one of the preceding Clauses, wherein at least a part of the PVDF material is configured as a band that is configured to at least partially cover an abdomen of a person.
10. A method of producing an ultrasound transducer, comprising:
   providing at least one ultrasound emitter comprising one or more lead zirconate titanate (PZT) crystals adapted to emit ultrasound energy into a desired area of interest; and
   providing at least one ultrasound receiver comprising a polyvinylildene fluoride (PVDF) material adapted to receive returned ultrasound signals echoed from the desired area of interest.
11. The method of Clause 10, wherein the PZT crystals are secured to the PVDF material.
12. The method of Clause 10 or Clause 11, wherein the PZT crystals are removably secured to the PVDF material.
13. The method of any one of Clauses 10 to 12, wherein the PZT crystals are woven into the PVDF material.
14. The method of any one of Clauses 10 to 13, further comprising:
   providing an excitation system adapted to excite the PZT crystals to emit said ultrasonic energy.
15. The method of any one of Clauses 10 to 14, wherein one or more of the PZT crystals are configured to be individually excited to emit said ultrasound energy.
16. The method of any one of Clauses 10 to 15, wherein one or more of the PZT crystals are configured to be simultaneously excited to emit said ultrasound energy.
17. The method of any one of Clauses 10 to 16, wherein at least a part of the PVDF material is configured as a band that is configured to at least partially surround an abdomen of a person.
18. The method of any one of Clauses 10 to 17, wherein at least a part of the PVDF material is configured as a band that is configured to at least partially cover an abdomen of a person.

## Claims

1. An ultrasound transducer (10), comprising:
at least one ultrasound emitter (12) comprising one or more lead zirconate titanate (PZT) crystals (18) adapted to emit ultrasound energy into a desired area of interest; and
at least one ultrasound receiver (20) comprising a polyvinylildene fluoride (PVDF) material (22) adapted to receive returned ultrasound signals echoed from the desired area of interest.

2. The ultrasound transducer (10) of Claim 1, wherein the PZT crystals (18) are secured and/or removably secured to the PVDF material (22).

3. The ultrasound transducer (10) of Claim 1 or Claim 2, wherein the PZT crystals (18) are woven into the PVDF material (22).

4. The ultrasound transducer (10) of any preceding Claims, further comprising:
an excitation system (28) adapted to excite the PZT crystals (18) to emit said ultrasonic energy.

5. The ultrasound transducer (10) of any preceding Claims, wherein one or more of the PZT crystals (18) are configured to be individually and/or simultaneously excited to emit said ultrasound energy.

6. The ultrasound transducer of any one of the preceding Claims, wherein one or more of the PZT crystals are configured to be simultaneously excited to emit said ultrasound energy.

7. The ultrasound transducer (10) of any preceding Claims, wherein at least a part of the PVDF material (22) is configured as a band (24) that is configured to at least partially surround and/or cover an abdomen (16) of a person (14).

8. A method of producing an ultrasound transducer (10), comprising:
providing at least one ultrasound emitter (12) comprising one or more lead zirconate titanate (PZT) crystals (18) adapted to emit ultrasound energy into a desired area of interest; and
providing at least one ultrasound receiver (20) comprising a polyvinylildene fluoride (PVDF) material (22) adapted to receive returned ultrasound signals echoed from the desired area of interest.

9. The method of Claim 8, wherein the PZT crystals (18) are secured and/or removably to the PVDF material (22).

10. The method of Claim 8 or Claim 9, wherein the PZT crystals (18) are woven into the PVDF material (22).

11. The method of any one of Claims 8 to 10, further comprising:
providing an excitation system (28) adapted to excite the PZT crystals (18) to emit said ultrasonic energy.

12. The method of any one of Claims 8 to 11, wherein one or more of the PZT crystals (18) are configured to be individually excited to emit said ultrasound energy.

13. The method of any one of Claims 8 to 12, wherein one or more of the PZT crystals are configured to be simultaneously excited to emit said ultrasound energy.

14. The method of any one of Claims 8 to 13, wherein at least a part of the PVDF material (22) is configured as a band (24) that is configured to at least partially surround and/or cover an abdomen (16) of a person (14).
